# EUROPÄISCHE PATENTANMELDUNG

(11) **EP 1 013 234 A2**
(43) Veröffentlichungstag der Anmeldung: **28.06.2000**
(21) Anmeldenummer: 99123218.2
(22) Anmeldetag: 25.11.1999
(51) Int. Cl.: A61B 19/02

(54) **Tragevorrichtung für Utensilien**

(30) Priorität: 26.11.1998 DE 19854674
(71) Anmelder: Rigling, Heinz, D-75382 Althengstett (DE)
(72) Erfinder:
(74) Vertreter: Dreiss, Fuhlendorf, Steimle & Becker

(57) **Zusammenfassung**

Eine Tragevorrichtung für an wechselnden Orten zu benutzende Utensilien, z. B. für Routineuntersuchungen am Krankenbett, weist einen ersten Tragebereich in einer im wesentlichen horizontalen Ausrichtung auf, der einen Fuß bildet, und einen zweiten Tragebereich mit einer Griffvorrichtung an seinem oberen Ende, der sich in bezug auf den Grundriß des ersten Tragebereichs überwiegend außermittig mit im wesentlichen vertikaler Ausrichtung bis in eine ein Mehrfaches der größten Ausdehnung des ersten Tragebereichs betragenden Höhe erstreckt. Auf den größeren der einander zugekehrten Seiten beider Tragebereiche sind wenigstens Plätze mit Halteelementen oder Ausgestaltungen für diese oder adapterartige Halterungen für ein Gehäuse aufweisende Utensilien (7, 8, 9), z. B. Meßgeräte, und/oder Plätze für behältnisartige Ausgestaltungen zur Aufnahme sonstiger Utensilien, z. B. Einmalartikel, vorgesehen.

## Beschreibung

Die Erfindung betrifft eine Tragevorrichtung für Utensilien, insbesondere für Utensilien für Kranken-Routineuntersuchungen am Krankenbett durch Pflegepersonal.

In Krankenhäusern z. B. muß das Pflegepersonal regelmäßig verschiedene Routineuntersuchungen bei den Kranken in den Krankenzimmern durchführen, wie z. B. Blutdruck-, Temperatur- und Blutzuckermessungen. Die hierzu erforderlichen Utensilien, das sind die jeweiligen Meßgeräte nebst Zubehör und Einmalartikel, wie z. B. Meßstreifen, Tupfer, Teststäbchen, Desinfektionsmittel usw., müssen vom Pflegepersonal jeweils in die Krankenzimmer mitgenommen werden, das sie hierzu, soweit möglich, in die Kitteltaschen steckt, ansonsten aber in den Händen trägt.

Dieses Tragen mit den Händen ist jedoch sehr hinderlich, so z. B. beim öffnen der Krankenzimmertüren. Andererseits können größere Utensilien, wie z. B. die Meßgeräte, aus den für ihr Einstecken nicht vorgesehenen Kitteltaschen herausfallen, wie auch eine Mitnahme von Einmalartikeln in ihnen immer wieder zum Suchen und Herumkramen darin führt.

Wegen des schwierigen Mitnehmens derartiger Utensilien werden z. B. oft mehrere Gänge hintereinander zu ein- und demselben Patienten gemacht, um so nach und nach alle Untersuchungen mit den dafür jeweils notwendigen Utensilien durchzuführen.
Um das Mitnehmen zu erleichtern, könnte zwar an ein Tablett gedacht werden, wie es z. B. benutzt wird, um die an die Patienten zu verabreichenden Spritzen in die Krankenzimmer mitzunehmen. Doch würde auch ein solches Tablett bei einer Bestückung mit allen benötigten Utensilien dazu führen, daß spontan keine Hand z. B. zum Öffnen der Krankenzimmertüren frei wäre, da es wegen des Gewichts und der Gefahr des Herunterfallens von Utensilien mit beiden Händen getragen werden müßte.

Ein weiterer Mißstand ist, daß es für diese Utensilien an geeigneten Ablagemöglichkeiten am Krankenbett während der Untersuchungen fehlt. Der jeweilige Nachttisch kann hierfür kaum benutzt werden, da seine sowieso sehr kleine Abstellfläche meist durch private Dinge des Patienten belegt ist und er auch zu weit vom Patienten entfernt ist, um z. B. während des Blutdruckmessens das mittels eines kurzen Schlauchs mit einer Druckmanschette verbundene Meßgerät darauf abzulegen. Dieses wird deshalb, wie die anderen erwähnten Meßgeräte auch, aufs Krankenbett gelegt, was unhygienisch ist.

Unbefriedigend ist auch, daß es für die im Zusammenhang mit der Durchführung der jeweiligen Untersuchungen anfallenden kontaminierten Abfälle keinen entsprechenden Sammelbehälter im Krankenzimmer gibt. Daher werden z. B. benutzte Blutzuckermeßstreifen, Tupfer für die Blutabnahme und die nach Beendigung der Temperaturmessung mit den heute üblichen digitalen Ohrthermometern zu entsorgenden sogenannten Meßhütchen kurzerhand in die üblicherweise am Nachttisch der Patienten hängenden Abfalltüten geworfen. Andererseits sollte ein dafür vorzusehender Sammelbehälter aus ästhetischen wie auch aus Sicherheitsgründen nach einer Benutzung nicht im Krankenzimmer verbleiben müssen.

Nach der Benutzung der erwähnten Meßgeräte-Utensilien kommt es immer wieder vor, daß eines davon beim Verlassen des Krankenzimmers beim Patienten vergessen wird. Dadurch besteht dann eine Gefahr der Entwendung, da die Krankenzimmer frei zugänglich sind und die Meßgeräte ohne weiteres auch privat zu Hause sinnvoll benutzt werden können. Auch werden erfahrungsgemäß Meßgeräte-Utensilien aus den Kitteltaschen des Pflegepersonals entwendet, bei denen es sich im Krankenhausbereich um kostspielige elektronisch-digitale Geräte handelt. Ihr Verlust durch Entwendung bedeutet daher einen erheblichen finanziellen Wiederbeschaffungsaufwand für die Krankenhäuser.

Der Erfindung lag daher das Problem zugrunde, eine Tragevorrichtung für an wechselnden Orten zu benutzende Utensilien zu schaffen, mit der insbesondere die geschilderten Mißstände in Krankenhäusern beseitigt werden können.

Gelöst wird das Problem mit einer Tragevorrichtung mit den Merkmalen des Anspruchs 1. vorteilhafte Ausgestaltungen der Erfindung sind Gegenstand der Unteransprüche.

Dadurch, daß die Tragevorrichtung eine gestellartige Ausgestaltung in einer einer Tragetasche ähnlichen Größenordnung aufweist, wobei ein erster Utensilien-Tragebereich eine im wesentlichen horizontale Ausrichtung aufweist und einen Vorrichtungsfuß bildet, ein zweiter Utensilien-Tragebereich sich in bezug auf den Grundriß des ersten Tragebereichs überwiegend außermittig mit im wesentlichen vertikaler Ausrichtung bis in eine ein Mehrfaches der größten Ausdehnung des ersten Tragebereichs betragenden Höhe erstreckt und eine Griffvorrichtung an seinem oberen Ende aufweist und zumindest im ersten Tragebereich im größeren seiner dem zweiten Tragebereich benachbarten Teilbereiche sowie im zweiten Tragebereich auf seiner diesem größeren Teilbereich zugewandten Seite wenigstens Plätze mit Halteelementen oder mit Ausgestaltungen zur Anordnung von Halteelementen oder adapterartigen Halterungen für ein Gehäuse aufweisende Utensilien und/oder Plätze für behältnisartige Ausgestaltungen zur Aufnahme sonstiger Utensilien vorgesehen sind, wird folgendes erreicht:
- die für die z. B. bei Krankenhaus-Patienten am Krankenbett für Routineuntersuchungen durch das Pflegepersonal, das andernorts auch ein ambulanter Pflegedienst sein kann, benötigten, normalerweise immer gleichen Utensilien können nun zur Mitnahme in die Krankenzimmer übersichtlich auf einer gemeinsamen Transportunterlage an immer derselben Stelle auf dieser angeordnet werden, was eine Vollständigkeitskontrolle bei den Gebrauchs- bzw. eine Verfügbarkeitskontrolle bei den Verbrauchsgegenständen vor dem Gang in die Krankenzimmer aber auch wieder vor deren Verlassen erleichtert, einen gezielten, schnellen Zugriff darauf ermöglicht und Zeitverlust durch zusätzliche Gänge, um vor oder nach dem Einsatz vergessene Utensilien zu holen oder auch zu suchen, vermeidet;
- das Mitnehmen von nicht wenigen und, wenn sie ein Gehäuse aufweisen, auch entsprechend Platz beanspruchenden Utensilien wird durch die dafür eine gemeinsame Transportunterlage bildende Tragevorrichtung in einer erfindungsgemäß immer noch handlichen Größenordnung, die z. B. der einer Tragetasche entspricht, vereinfacht und erleichtert, da hierdurch ein bequemes, körpernahes Tragen mit nur einer Hand am ungezwungen in natürlicher Haltung herunterhängenden Arm, also ohne ermüdende Anstrengung, möglich ist; dazu trägt auch die außermittige Anordnung des zweiten Tragebereichs in bezug auf den ersten bei, wodurch auch die ein Gehäuse aufweisenden, sozusagen sperrigen Utensilien auf denjenigen der einander zugekehrten Trage-Teilbereiche der Tragevorrichtung angeordnet werden können, die sich beim Tragen der Tragevorrichtung auf der körperabgewandten Seite der diese tragenden Person befinden können, wo sie folglich dieses Tragen nicht behindern;
- diese ein Gehäuse aufweisenden und folglich schwereren Utensilien können überwiegend auf dem breiteren der beiden Teilbereiche des ersten Tragebereichs, die demgegenüber leichteren sonstigen Utensilien oberhalb von diesen in behältnisartigen Ausgestaltungen im zweiten Tragebereich angeordnet werden, was sich beim Abstellen der erfindungsgemäß asymmetrisch gestalteten Tragevorrichtung vorteilhaft auf deren Standsicherheit auswirkt; dabei kann eine solche, die unterschiedlichen Größen der ein Gehäuse aufweisenden Utensilien berücksichtigende Anordnung der behältnisartigen Ausgestaltungen im zweiten Tragebereich kompakte Ausmaße der Tragevorrichtung ähnlich derjenigen einer Tragetasche ermöglichen;
- durch die ein Tragen mit einer Hand ermöglichende Vorrichtungs-Ausgestaltung ist außerdem die andere Hand frei, um z. B. Türen zu öffnen wie auch, z. B. im Krankenhaus, den Personal-Anwesenheits-Kontrollschalter nach dem Betreten des Krankenzimmers zu betätigen;
- das Bereithalten der z. B. bei Patienten benötigten Utensilien in der Nähe des Krankenbetts wird erleichtert, da die Tragevorrichtung mit den darauf angeordneten Utensilien mittels ihres ersten, einen Vorrichtungsfuß bildenden Tragebereichs auf einer Unterlage in der Nähe vom Krankenbett im Bereich der Nähe des Patienten-Oberkörpers, also dort, wo die Utensilien eingesetzt werden, abgestellt werden kann, z. B. auf einem der üblicherweise beidseits am Nachttisch vorhandenen abklappbaren Borde; somit besteht keine Notwendigkeit mehr, etwa das Krankenbett oder die meist überladene Platte des Nachttischs als Ablage für diese Utensilien benutzen zu müssen;
- die vorgesehene Anordnung der Utensilien in unmittelbarer Nähe zueinander auf einer gemeinsamen Transport- und damit auch Haltevorrichtung ermöglicht z. B. auch das zeitsparende gleichzeitige Benutzen von zwei dieser Utensilien, von denen eines ggf. sogar zur Benutzung auf der Tragevorrichtung fixiert bleiben kann, im Krankenhaus z. B. eines der heute üblichen digitalen Blutdruckmeßgeräte, während ein anderes zur parallelen Benutzung zu diesem von der Tragevorrichtung abgenommen wird, im Krankenhaus z. B. eines der heute zum Messen der Körpertemperatur benutzten digitalen Ohrthermometer;
- mittels der im Hinblick auf die verschiedenen mitzunehmenden Utensilien entsprechend unterschiedlich ausgestalteten Utensilien-Unterbringungsmöglichkeiten auf der Tragevorrichtung, nämlich behältnisartige Ausgestaltungen für die besagten Einmal- bzw. Verbrauchsartikel, die darin zum schnellen Zugriff lose eingelegt werden können, und Plätze mit Halteelementen bzw. Halterungen für die ein Gehäuse aufweisenden Utensilien, können sämtliche Utensilien verliersicher, also ohne Gefahr von Beschädigungen durch Herunterfallen, mitgenommen werden, was das Mitnehmen vereinfach und erleichtert;
   für die wertvolleren darunter, das sind die ein Gehäuse aufweisenden Utensilien, wie z. B. die erwähnten Meßgeräte im Krankenhaus, können in bezug auf ihre vorgesehene Anordnung auf der erfindungsgemäß gestellartig und damit stabilen Tragevorrichtung aber nicht nur verliersicher, sondern auch - in bezug auf die Tragevorrichtung - entwendungshemmend auf dieser angeordnet werden, was ihre Entwendung im Vergleich zu einzelnen, ohne Zusammenhalt durch eine gemeinsame Transportunterlage untereinander lose mitgenommenen Utensilien zumindest erschwert und so die z. B. in Krankenhäusern bisher durch Entwendung bedingten kostenträchtigen Ersatzbeschaffungen erheblich gesenkt werden können;
- das Vorsehen von z. B. adapterartigen Halterungen im ersten Tragebereich ermöglicht das Umrüsten einer vorhandenen Tragevorrichtung auf neue, ebenfalls ein Gehäuse aufweisende Utensilien, da hierfür u. U. nur ein in die vorhandene Adaptierung passender Adapter benötigt wird, also keine Veränderungen an der Tragevorichtung selbst vorgenommen werden müssen; dadurch läßt sich auch die Herstellung der Tragevorrichtung vereinfachen und verbilligen, da so der erste Tragebereich nicht zur Anordnung von bestimmten, momentan gebräuchlichen Utensilien mit einem Gehäuse, sondern nur von änderbaren Adaptern vorbereitet werden kann;
- die Tragevorrichtung in der vorgesehenen Größenordnung einer Tragetasche kann, z. B. für eine Benutzung im Krankenhaus, auf dem größeren der Teilbereiche des ersten Tragebereichs auch z. B. Platz für einen Abfall-Sammelbehälter bieten, also für ein weiteres Utensil mit einem Gehäuse, in den die zuvor erwähnten benutzten Einmalartikel eingeworfen werden können; dieser Behälter kann sich durch eine Anordnung auf einer solchen Tragevorrichtung nun ohne zusätzlichen Aufwand immer mit den übrigen benötigten Utensilien am jeweiligen Ort der Abfallentstehung, insbesondere also am Krankenbett, befinden, was dann das Abfallsammeln für eine sachgerechte Entsorgung unmittelbar beim Patienten ermöglicht, wobei dieser Behälter durch die mögliche verliersichere Anordnung auf der Tragevorrichtung auch nicht im Krankenzimmer vergessen werden kann;
- da es bei der vorgesehenen Größe der Tragevorrichtung ausreichend sein kann, die behältnisartigen Ausgestaltungen zur Aufnahme von Utensilien nur auf einer Seite des zweiten Tragebereichs vorzusehen, nämlich derjenigen, die zum größeren Teilbereich des ersten Tragebereichs weist, können mithin auf deren Rückseite weitere, vorzugsweise wenig ausladende Ausgestaltungen vorgesehen werden, die das körpernahe Tragen der Tragvorrichtung nicht behindern, wie z. B. eine schmale Vorrichtung zum Aufhängen der Tragevorrichtung an einer dies ermöglichenden Einrichtung.

Für eine kostengünstige Herstellung der Tragevorrichtung können vorteilhaft sowohl der erste als auch der zweite Tragebereich jeweils die Form einer im wesentlichen rechteckigen Trageplatte aufweisen, wobei die zweite Trageplatte im Hinblick auf die vorgesehene Anordnung der schwereren und größeren, ein Gehäuse aufweisenden Utensilien auf der ersten Trageplatte mit Vorteil auf der ersten Trageplatte in einer in dieser außermittig ausgebildeten Nut senkrecht angeordnet ist.

Für ein bequemes, kofferähnliches Tragen der Tragevorrichtung mit einer Hand seitlich nahe am Körper der sie tragenden Person hat die erste Trageplatte vorzugweise eine Breite von lediglich annähernd zwei Handbreit und eine etwa doppelt so große Länge.

Damit die zweite Trageplatte genügend Platz für die vorgesehenen behältnisartigen Ausgestaltungen zur Aufnahme von Utensilien aufweist, ist sie vorzugsweise so breit, wie die erste Trageplatte lang ist.

Weitere Einzelheiten, Merkmale und Vorteile der Erfindung ergeben sich aus der nachfolgenden Beschreibung unter Bezugnahme auf die schematischen Zeichnungen, in denen ein ein bevorzugtes Ausführungsbeispiel der Erfindung dargestellt ist. Es zeigen:

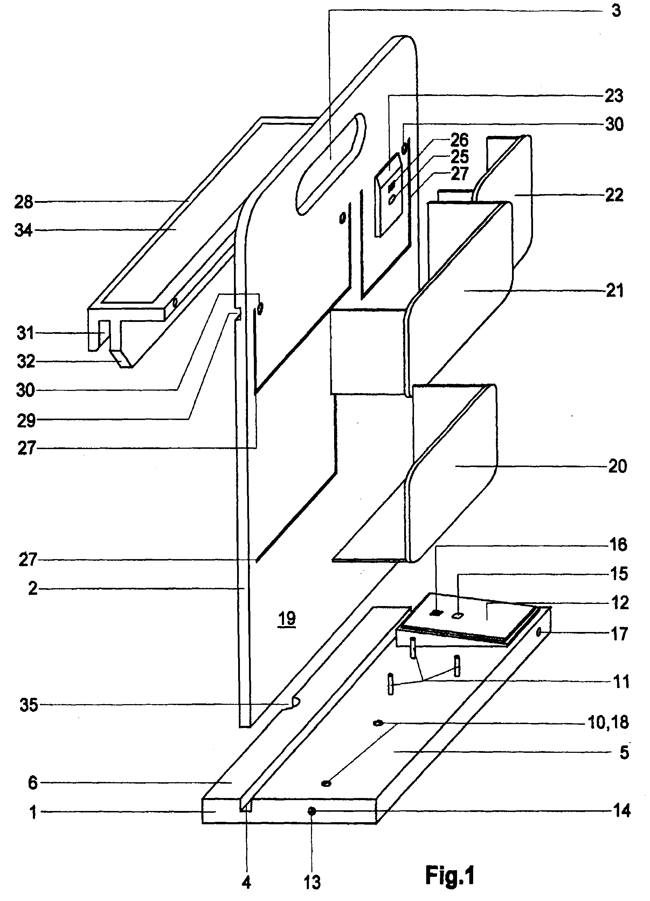

- Fig. 1: in einer perspektivischen Ansicht in Explosionsdarstellung den Aufbau einer hier zur Mitnahme von Utensilien für Kranken-Routineuntersuchungen am Krankenbett durch Pflegepersonal bestimmten Tragevorrichtung und
- Fig. 2: die Tragevorrichtung nach Fig. 1 nach der Bestückung mit ein Gehäuse aufweisenden Utensilien.

Die in den beiden Fig. gezeigte Utensilien-Tragevorrichtung hat einen gestellartigen Aufbau aus einer ersten und einer zweiten rechteckigen Utensilien-Trageplatte 1 und 2 aus einem leichten Werkstoff wie z. B. Aluminium oder Kunststoff. Die als Vorrichtungsfuß zum Abstellen der Tragevorrichtung auf einer Unterlage vorgesehene Trageplatte 1 ist dementsprechend horizontal, die Trageplatte 2 dagegen vertikal ausgerichtet. Sie hat am oberen Ende eine Grifföffnung 3.

Im Hinblick auf die bei diesem gezeigten Ausführungsbeispiel einer Tragevorrichtung zur Anordnung auf der ersten Trageplatte 1 vorgesehenen Utensilien mit einem Gehäuse ist diese ca. 16 cm breit und etwa doppelt so lang und die zweite Trageplatte 2 daran angepaßt ebenfalls ca. 32 cm breit, aber ca. 46 cm lang bzw. hoch, was ein bequemes seitliches Tragen der Tragevorrichtung nahe am Körper mit nur einer Hand, z. B. nach Art einer Tragetasche oder eines Aktenkoffers, ermöglicht.

Auf der Oberseite der ersten Trageplatte 1 ist in Längsrichtung eine Nut 4 extrem außermittig vorgesehen, d. h. weit nach links versetzt. Darin ist die zweite Trageplatte 2 senkrecht eingesetzt und befestigt, z. B. eingeklebt oder von der Unterseite der ersten Trageplatte 1 her lediglich verschraubt, um sie ggf. zur Anpassung an sich gehäusernäßig ändernde Utensilien leicht austauschen zu können. Die Nut 4 bzw. die Trageplatte 2 unterteilt die erste Trageplatte 1 in einen sehr breiten Teilbereich 5 und einen sehr schmalen Teilbereich 6.

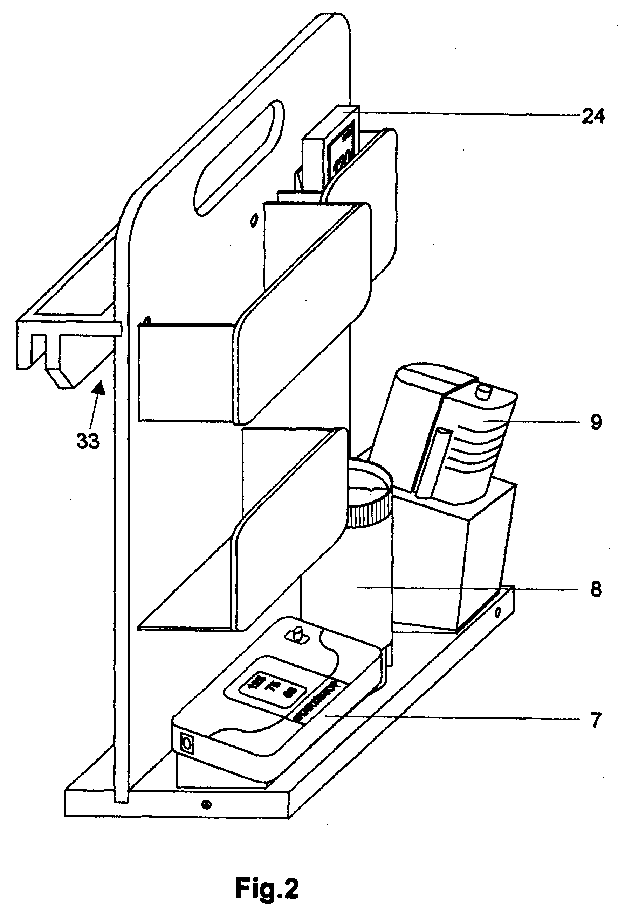

Der breite Teilbereich 5 der Trageplatte 1 bietet, wie in Fig. 2 gezeigt, Platz zur Anordnung von drei Utensilien mit einem Gehäuse, und zwar hier für ein digitales Blutdruckmeßgerät 7, einen Abfall-Sammelbehälter 8 und ein digitales Ohrthermometer 9, wobei sich die Plazierung dieser schwereren Utensilien auf diesem Teilbereich 5 vorteilhaft auf die Standfestigkeit der Tragevorrichtung im Hinblick auf die außermittige Anordnung der Trageplatte 2 auswirkt.

Diese drei Utensilien 7, 8, 9 sind auf unterschiedliche Weise verliersicher auf der Trageplatte 1 befestigt. Für das Blutdruckmeßgerät 7 sind hierfür zwei Löcher 10 in der Trageplatte 1 vorgesehen, in die zwei entsprechend an der Unterseite des Gehäuses des Blutdruckmeßgeräts 7 - oder auch an z. B. einer entsprechenden Gehäuse-Adapterplatte - angeordnete Haltestifte eingesteckt werden können. Für den in Fig. 2 gezeigten zylindrischen Abfall-Sammelbehälter 8 sind drei in der Trageplatte 1 angeordnete Haltestifte 11 und für das Ohrthermometer 9 ist ein Haltesockel 12 auf der Trageplatte 1 befestigt, der im Hinblick auf die oberhalb davon auf der Trageplatte 2 vorgesehenen Ausgestaltungen schräg nach hinten ansteigend ausgeführt ist, damit das zur Benutzung von der Tragevorrichtung abzunehmende Ohrthermometer 9 leicht von dem Sockel 12 abgenommen werden kann.

Der Abfall-Sammelbehälter 8 kann statt mittels der drei Stifte 11 auch z. B. mittels eines auf der Trageplatte 1 angeordneten Adapters gehalten werden, auf dem er z. B. mit seinem Boden lösbar befestigt werden kann. Es kann hierfür aber auch eine an der Trageplatte 2 befestigte adapterartige Haltevorrichtung vorgesehen werden, wie sie z. B. aus dem Gebrauchsmuster DE 297 20 424 U1 bekannt ist.

Damit das kostspielige digitale Blutdruckmeßgerät 7 nicht nur sicher gegen Herunterfallen von der Trageplatte 1 auf dieser befestigt, sondern trotz seiner durch die vorgesehenen Haltestife grundsätzlich leichten Entfernbarkeit von der Trageplatte 1 seine Entwendung von der Tragevorrichtung zumindest erschwert ist, ist in einer Bohrung 13 mit Innengewinde eine Zylinderschraube 14 eingedreht, die z. B. in eine entsprechende Ausnehmung im betreffenden Gehäuse-Haltestift dieses Geräts 7 eingreift. Damit diese Schraube 14 nicht ohne weiteres herausgedreht werden kann, ist ihr Kopf z. B. mit einem ein Spezialwerkzeug erfordernden 3 -fach-Sternschlitz versehen.

Um auch die Entwendung des Ohrthermometers 9 von der Tragevorrichtung zumindest zu hemmen, das zu seiner Benutzung jeweils vom Haltesockel 12 abgenommen werden muß, ist in diesem eine Ohrthermometer-Vorhandensein-Überwachungsvorrichtung eingebaut. Sie umfaßt hier z. B. einen opto-elektronischen ÜberwachungsSensor 15, der zur Ausbildung einer Reflex-Lichtschranke mit dem Boden des Gehäuses des Ohrthermometers 9 zusammenwirkt, und einen akustischen Alarmgeber 16. Dieser wird z. B. zeitgesteuert automatisch aktiviert, wenn das Ohrthermometer 9 länger als die für eine Temperaturmessung übliche kurze Zeitdauer, das ist normalerweise weniger als eine Minute, vom Sockel 12 abgenommen ist.

Zur Stromversorgung der Vorhandensein-Überwachungsvorrichtung des mit Batterien betriebenen Ohrthermometers 9 sowie z. B. auch des Blutdruckmeßgeräts 7 ist unterhalb des Sockels 12, z. B. in einer Ausnehmung auf der Unterseite der Trageplatte 1, vorzugsweise ein Akku angeordnet, für dessen Ladezustandskontrolle ein Kontrollicht 17 an der Stirnseite der Trageplatte 1 vorgesehen ist. Vom Akku kann der Strom z. B. über in die Trageplatte 1 eingegossene Leitungen zu in den Löchern 10 angeordneten metallenen Buchsen 18 und über die darin einzusteckenden ebenfalls metallenen Gehäse-Haltestifte des Blutdruckmeßgeräts 7 in dieses geleitet werden. Dieses Gerät 7 wäre somit bei einer evtl. Entwendung von der Tragevorrichtung mangels eigener Stromversorgung unbrauchbar, was ebenfalls einer Entwendung, z. B. auch durch das Pflegepersonal, denen dies bekannt ist, entgegenwirkt.

Würde statt des Akkus eine Batterie vorgesehen werden, so könnte diese, ohne daß das dann auch von ihr versorgte Blutdruckmeßgerät 7 erst umständlich von der ersten Trageplatte 1 abgenommen werden müßte, erneuert werden.

Auf der dem breiten Teilbereich 5 der Trageplatte 1 zugewandten Seite 19 der Trageplatte 2 sind drei behältnis- oder fachartige Ausgestaltungen 20, 21 und 22 zur Aufnahme weiterer Utensilien vorgesehen. Im Krankenhausbereich kann es sich bei diesen einmal um die - nicht gezeigte - Druckmanschette des Blutdruckmeßgeräts 7 handeln, für deren Aufbewahrung während des Nichtgebrauchs das untere Fach 20 vorgesehen ist, das hierzu nicht nur oben, sondern auch seitlich zum linken Rand der Trageplatte 2 hin offen ist, um die Druckmanschette darin ohne großen Zeitaufwand ablegen und auch leicht wieder herausnehmen zu können. Über dem Druckmanschettenfach 20 befindet sich ein zweites, längeres, nur oben offenes Fach 21, das hier zum lose Einlegen von Einmal- sowie Verbrauchsartikeln, wie z. B. Blutzucker-Meßstreifen, Tupfer, Desinfektionsmittel usw. vorgesehen ist.

Rechts daneben befindet sich ein drittes schmales und kurzes Fach 22, in dem ein sockelartiges Gehäuse 23 auf der Trageplatte 2 befestigt ist. Es ist zum leichten Einstecken des Blutzuckermeßgeräts 24 in das Fach 22 an seinem oberen Ende abgeschrägt.

Um auch die Entwendung dieses kostspieligen digitalen Meßgeräts 24 von der Tragevorrichtung zu erschweren, ist in dem Gehäuse 23 ebenfalls eine Vorhandensein-Überwachungsvorrichtung mit einem opto-elektronischen Sensor 25 untergebracht, die auch zeitgesteuert einen akustischen Alarmgeber 26 aktiviert, wenn das Blutzuckermeßgerät 24 länger als die für diese Messungungen übliche Zeitdauer, hier bis zu mehrere Minuten, aus dem Fach 22 herausgenommen ist.

Diese Überwachungsvorrichtung des ebenfalls mit Batterien betriebenen Meßgeräts 24 kann zur Stromversorgung auch mit dem zuvor erwähnten Akku in der Trageplatte 1 verbunden sein, z. B. über in entsprechende Nuten in der Trageplatte 2 eingegossene Leitungen.

Statt mit einem eigenen Alarmgeber 26 ausgestattet zu sein, kann diese Überwachungsvorrichtung auch mit dem Alarmgeber 16 der Vorhandensein-Überwachungsvorrichtung des Ohrthermometers 9 zusammenarbeiten.

Die drei Fächer 20, 21, 22 sind hier kostengünstig jeweils von einer Art unvollständigem Rahmengerüst gebildet, das optisch ansprechend stirnseitig von einer ringsum in vertikaler Richtung darüber überstehenden, z. B. auch andersfarbig gestalteten Abdeckung begrenzt ist. Für eine sichere, einfach auszuführenden Befestigung dieser Rahmengerüste an der Trageplatte 2 können in dieser z. B. entsprechende Nuten 27 ausgebildet sein, in die die Rahmen eingesetzt und z. B. darin verleimt sind. Auf der Rückseite der zweiten Trageplatte 2 ist in deren oberem Endbereich eine Tragevorrichtungs-Halterung 28 in Form einer Profilschiene in eine dort dafür vorgesehene Nut 29 eingesetzt und für einen festen Halt mit Hilfe von durch Bohrungen 30 geführte Schrauben mit der Trageplatte 2 verschraubt.

Diese Halterung 28 weist eine Nut 31 auf, die z. B. auf eine der z. B. in Krankenhäusern für solche Zwecke üblicherweise im Kopfbereich der Krankenbetten montierte Normschiene mit einem Querschnitt von 25 x 10 mm paßt, um so die Tragevorrichtung z. B. für die Dauer der Benutzung der auf ihr befindlichen Utensilien in Bett-Nähe aufhängen zu können. Davon abgesehen kann die Tragevorrichtung durch den zwischen der Trageplatte 2 und der nach unten verlängerten inneren Nutwange 32 vorhandenen Zwischenraum 33 z. B. auch auf den oberen Holm des Fußteils des Bettgestells oder auch am mit einer entsprechenden Haltestange versehenen Nachttisch bzw. an der Griffstange einer seiner Schubladen aufgehängt werden, wofür diese Nutwange zum leichteren Einführen seitlich abgeschrägt ist. Zur Durchführung der Routineuntersuchungen ist dann der Nachttisch ggf. näher ans Krankenbett heranzuziehen.

Auf der Oberseite dieses Halterungsprofils 28 kann ein Solarmodul 34 mit einer oder mehreren Solarzellen zum Laden des ggf. in der Trageplatte 1 unterbrachten Akkus vorgesehen werden. Es können aber auch in dem Halterungsprofii 28 z. B. elektrische Kontaktelemente vorgesehen werden, um die Tragevorrichtung während ihres Nichtgebrauchs, z. B. in einem entsprechenden Raum, auf einer Art Serviceschiene mit entsprechend komplementär ausgebildeten Kontaktelementen aufzuhängen, die mit einem Akku-Ladegerät verbunden sind, um so z. B. den TragevorrichtungsAkku aufzuladen.

Um ggf. während des moblilen Einsatzes der Tragevorrichtung längere Auflade-Pausen des Akkus zu vermeiden, wenn die Tragevorrichtung vorübergehend nicht im Bereich einer Lichtquelle abgestellt ist, kann eine ebenfalls zeitgesteuert automatisch aktivierte elektro-akustische Alarmeinrichtung vorgesehen werden. Diese könnte z. B. auch die Alarmgeber der beiden Vorhandensein-Überwachungsvorrichtungen ersetzen.

Die Halterungsschiene 28 kann auch benutzt werden, um die Tragevorrichtung z. B. an einem fahrbaren Infusionsständer oder sonstigen Stativ aufzuhängen. Für diesen Fall kann eine etwa halbkreisförmige Aussparung 35 in der Stirnseitenmitte des schmalen Teilbereichs 6 der Trageplatte 1 vorgesehen werden, die das senkrechte Ständer- bzw. Stativ-Rohr teilweise umgreift, um so ein Hin- und Herpendeln der Tragevorrichtung beim Fahren des Ständers oder Stativs zu vermeiden.

Um die auf der Tragevorrichtung mitgenommenen Utensilien z. B. vor Nässe oder sonstige Einflüssen zu schützen, kann eine an ihrem oberen Ende offene Haube vorgesehen werden, die bis unter die Grifföffnung 3 auf die Tragevorrichtung aufgezogen wird. Um sie bei Nichtgebrauch an der Tragevorrichtung verstauen zu können, kann auf der Rückseite der Trageplatte 2 z. B. eine entsprechende Tasche oder dergl. vorgesehen werden.

Auch können auf dieser Rückseite z. B. Halterungen für Schreibutensilien vorgesehen werden.

## Patentansprüche

1. Tragevorrichtung für Utensilien, insbesondere für Utensilien für Kranken-Routineuntersuchungen am Krankenbett durch Pflegepersonal, in einer gestellartigen Ausgestaltung in einer einer Tragetasche ähnlichen Größenordnung, wobei
- ein erster Utensilien-Tragebereich (1) eine im wesentlichen horizontale Ausrichtung aufweist und einen Vorrichtungsfuß bildet,
- ein zweiter Utensilien-Tragebereich (2) sich in bezug auf den Grundriß des ersten Tragebereichs (1) überwiegend außermittig mit im wesentlichen vertikaler Ausrichtung bis in eine ein Mehrfaches der größten Ausdehnung des ersten Tragebereichs (1) betragenden Höhe erstreckt und eine Griffvorrichtung (3) an seinem oberen Ende aufweist und
- zumindest im ersten Tragebereich (1) im größeren (5) seiner dem zweiten Tragebereich (2) benachbarten Teilbereiche (5, 6) sowie im zweiten Tragebereich (2) auf seiner diesem größeren Teilbereich (5) zugewandten Seite (19) wenigstens Plätze mit Halteelementen (11) oder mit Ausgestaltungen (10) zur Anordnung von Halteelementen oder adapterartigen Halterungen (12) für ein Gehäuse aufweisende Utensilien (7, 8, 9) und/oder Plätze für behältnisartige Ausgestaltungen (20, 21,22) zur Aufnahme sonstiger Utensilien vorgesehen sind.

2. Tragevorrichtung nach Anspruch 1, dadurch gekennzeichnet, daß der erste Tragebereich (1) als eine rechteckige Trageplatte mit einer Breite von annähernd zwei Handbreit und einer etwa doppelt so großen Länge ausgebildet ist.

3. Tragevorrichtung nach Anspruch 1 oder 2, dadurch gekennzeichnet, daß der zweite Tragebereich (2) als eine rechteckige Trageplatte ausgebildet ist.

4. Tragevorrichtung nach Anspruch 2 und 3, dadurch gekennzeichnet, daß die Breite der zweiten Trageplatte (2) der Länge der ersten Trageplatte (1) entspricht.

5. Tragevorrichtung nach Anspruch 2 und 3 oder nach Anspruch 4, dadurch gekennzeichnet, daß die erste Trageplatte (1) eine außermittig in Richtung ihrer Längsausdehnung randparallel verlaufende Nut (4) aufweist, die sie in einen schmalen (6) und einen demgegenüber sehr viel breiteren Teilbereich (5) unterteilt, und daß die zweite Trageplatte (2) senkrecht zur ersten Trageplatte (1) in dieser Nut (4) angeordnet ist.

6. Tragevorrichtung nach Anspruch 5, dadurch gekennzeichnet, daß im sehr viel breiteren Teilbereich (5) der ersten Trageplatte (1) in Längsrichtung aufeinanderfolgend drei Plätze zur Anordnung eines digitalen Blutdruckmeßgeräts (7), eines Abfall-Sammelbehälters (8) und eines digitalen Ohrthermometers (9) unter Berücksichtigung des Platzbedarfs ihrer jeweiligen Gehäuse in bezug auf die an der zweiten Trageplatte vorgesehenen Ausgestaltungen (20, 21, 22) vorgesehen sind.

7. Tragevorrichtung nach Anspruch 5 oder 6, dadurch gekennzeichnet, daß auf der dem sehr viel breiteren Teilbereich (5) zugewandten Seite (19) der zweiten Trageplatte (2) eine Anzahl behältnisartiger Ausgestaltungen (20, 21, 22) vorgesehen sind, die unter Berücksichtigung der Gehäusegröße der zur Anordnung auf der Trageplatte 1 vorgesehenen Utensilien (7, 8, 9) über- und nebeneinander angeordnet in unterschiedlicher Breite, Tiefe und Höhe fachartig von ihr seitwärts abstehen.

8. Tragevorrichtung nach Anspruch 7, dadurch gekennzeichnet, daß eine untere (20) und zwei obere (21, 22) behältnisartige Ausgestaltungen vorgesehen sind, wobei eine der oberen zur Aufnahme eines digitalen Blutzuckermeßgeräts (24) und die untere zur Aufnahme der Druckmanschette des Blutdruckmeßgeräts (7) bestimmt ist, die hierzu auch zum benachbarten Seitenrand der zweiten Trageplatte (2) hin offen ist.

9. Tragevorrichtung nach Anspruch 6, dadurch gekennzeichnet, daß die Ausgestaltung (10) zur Anordnung von Halteelementen für das Blutdruckmeßgerät (7) Anpassungen (18) für eine Einspeisung von elektrischem Strom über die Halteelemente in dieses Gerät (7) aufweisen.

10. Tragvorrichtung nach Anspruch 6 und 9, dadurch gekennzeichnet, daß für das Ohrthermometer (9) als adapterartige Halterung (12) ein Haltesockel vorgesehen ist.

11. Tragevorrichtung nach Anspruch 10, dadurch gekennzeichnet, daß in den Haltesockel (12) zumindest eine VorhandenseinÜberwachungseinrichtung für das Ohrthermometer (9) integriert ist.

12. Tragevorrichtung nach Anspruch 11, dadurch gekennzeichnet, daß auch für das Blutzuckermeßgerät (24) eine Vorhandensein-Überwachungseinrichtung vorgesehen ist.

13. Tragevorrichtung nach Anspruch 8 und 12, dadurch gekennzeichnet, daß diese Vorhandensein-Überwachungseinrichtung in einem in der betreffenden behältnisartigen Ausgestaltung (22) angeordneten Gehäuse (23) enthalten ist.

14. Tragevorrichtung nach Anspruch 11, 12 oder 13, dadurch gekennzeichnet, daß beide Vorhandensein-Überwachungsvorrichtungen zumindest einen elektro-akustischen Alarmgeber (16, 26) sowie ein opto-elektronisches Überwachungselement (15, 25) aufweisen.

15. Tragevorrichtung nach einem der vorhergehenden Ansprüche 6 bis 12, dadurch gekennzeichnet, daß als Stromquelle für das Blutdruckmeßgerät(7) und die beiden VorhandenseinÜberwachungseinrichtungen ein Akku vorgesehen ist.

16. Vorrichtung nach Anspruch 9, dadurch gekennzeichnet, daß das größere (21) der beiden oberen Fächer (21, 22) zur Aufnahme von Einmal-Artikeln, wie z. B. Tupfern, Teststäbchen, Desinfektionsmitteln, und das untere Fach (20) zur Aufnahme der mit dem Blutdruckmeßgerät (7) verbundenen Druckmanschette vorgesehen ist.

17. Tragevorrichtung nach einem der Ansprüche 2 bis 14 dadurch gekennzeichnet, daß zumindest die erste Trageplatte (1) zumindest in Teilbereichen hohl ausgebildet ist.

18. Tragevorrichtung wenigstens nach einem der Ansprüche 5 bis 15, dadurch gekennzeichnet, daß auf der Rückseite der zweiten Trageplatte (2) ein Mittel (28) zum Auf- oder Einhängen der Tragevorrichtung vorgesehen ist.

19. Tragevorrichtung nach Anspruch 16, dadurch gekennzeichnet, daß als Mittel (28) ein waagerecht an der zweiten Trageplatte (2) angeordnetes schienenförmiges Halterungsprofil vorgesehen ist.

20. Tragevorrichtung wenigstens nach Anspruch 13 und 17, dadurch gekennzeichnet, daß auf dem Halterungsprofil (28) eine oder mehrere Solarzellen (34) vorgesehen sind.
